# EUROPÄISCHE PATENTSCHRIFT

(11) **EP 2 890 330 B1**
(45) Veröffentlichungstag und Bekanntmachung des Hinweises auf die Patenterteilung: **22.03.2017**
(21) Anmeldenummer: 13753313.9
(22) Anmeldetag: 23.08.2013
(51) Int. Cl.: A61F 2/00

(54) **KÜNSTLICHER SPHINKTER**
ARTIFICIAL SPHINCTER
SPHINCTER ARTIFICIEL

(30) Priorität: 28.08.2012 DE 102012215243
(43) Veröffentlichungstag der Anmeldung: 08.07.2015
(73) Patentinhaber: Dualis Medtech GmbH, 82229 Seefeld (DE)
(72) Erfinder: SEEBACH, Michael, 80805 München (DE); DUDZIAK, Marco, 97483 Eltmann (DE); MICHEL, Sören, 82229 Seefeld (DE); STEER, Christian, 82234 Weßling OT Oberpfaffenhofen (DE)
(74) Vertreter: dompatent von Kreisler Selting Werner - Partnerschaft von Patent- und Rechtsanwälten mbB
(86) Internationale Anmeldenummer: PCT/EP2013/067544
(87) Internationale Veröffentlichungsnummer: WO 2014/033062

(56) Entgegenhaltungen:
- WO-A2-01/45488
- US-A- 3 863 622
- US-A1- 2005 240 144
- US-A1- 2010 211 175
- US-A1- 2010 256 757
- US-A1- 2012 157 759

## Beschreibung

Die Erfindung betrifft einen künstlichen Sphinkter zum steuerbaren Okkludieren eines harnleitenden Gefäßes bei einem inkontinenten Patienten. Die Erfindung betrifft ferner ein Verfahren zum Steuern eines künstlichen Sphinkters.

Es ist bekannt, inkontinenten Patienten einen künstlichen Sphinkter zu implantieren. Ein derartiges System wird beispielsweise von der Firma American Medical Systems unter der Bezeichnung AMS 800 angeboten. Hierbei handelt es sich um ein rein hydraulisch aktuiertes System, bei dem eine hydraulisch betätige Okklusionsvorrichtung um ein harnleitendes Gefäß des Patienten angeordnet wird. Die Okklusionsvorrichtung ist über eine Leitung mit einem Reservoir verbunden. Die Okklusionsvorrichtung wird mit dem Hydraulikfluid aus dem Reservoir gefüllt und verschließt hierdurch das durch sie hindurch laufende harnleitende Gefäß. Zwischen dem Reservoir und der Okklusionsvorrichtung ist eine manuell betätigbare Pumpe angeordnet, die derart im Körper des Patienten implantiert wird, dass sie von außen betätigbar ist. Durch Betätigen dieser Pumpe wird Hydraulikfluid von der Okklusionsvorrichtung in das Reservoir zurückgefördert, sodass die Okklusionsvorrichtung geöffnet wird und der Patient seine Blase entleeren kann.

Nachteilig an dem beschriebenen System ist, dass dieses auf einen bestimmten Maximaldruck ausgelegt werden muss, der ausreicht, um das harnleitende Gefäß auch während sogenannter Stresssituationen sicher zu verschließen. Eine Stresssituation tritt auf, wenn der Druck in dem harnleitenden Gefäß durch verschiedene Umstände ansteigt. Dies kann beispielsweise passieren, wenn der Patient hustet, niest oder sich in bestimmter Weise körperlich betätigt. Während solcher Situationen kann es zu einem kurzzeitigen sehr starken Druckanstieg in dem harnleitenden Gefäß kommen. Auch in diesen Situation muss das harnleitende Gefäß durch den künstlichen Sphinkter sicher verschlossen werden, sodass es bei dem beschriebenen System notwendig ist, den Sphinkter konstant mit einem relativ hohen Druck zu beaufschlagen. Eine dauerhafte Beaufschlagung des harnleitenden Gefäßes mit einem hohen Druck führt häufig zu einer Beschädigung des Gefäßes, beispielsweise durch Atrophie.

Weiterhin kann das beschriebene System nur in sehr beschränktem Ausmaß an Veränderungen im Körper des Patienten angepasst werden. So kann es nach einer Implantation notwendig sein, das harnleitende Gefäß mit einem höheren oder niedrigeren Druck zu verschließen als ursprünglich vorgesehen war. Eine flexible Anpassung des Verschlussdrucks kann bei dem beschriebenen System nur dadurch erreicht werden, dass dem System z. B. über einen Port zusätzliches Hydraulikfluid zugeführt wird. Dies bedeutet einen invasiven Eingriff für den Patienten.

Aufgabe der Erfindung ist es, einen künstlichen Sphinkter bereitzustellen, durch den ein harnleitendes Gefäß auch in Stresssituationen sicher verschlossen werden kann, wobei die Gefahr einer dauerhaften Schädigung des harnleitenden Gefäßes minimiert wird. Die Erfindung soll ferner ein entsprechendes Verfahren zum Steuern eines künstlichen Sphinkters bereitstellen.

Die Lösung der Aufgabe erfolgt erfindungsgemäß durch die Merkmale der Ansprüche 1 und 7.

Der erfindungsgemäße künstliche Sphinkter dient dem steuerbaren Okkludieren eines harnleitenden Gefäßes bei einem inkontinenten Patienten. Der Sphinkter weist eine implantierbare insbesondere ringförmige hydraulische Okklusionsvorrichtung auf, die in implantiertem Zustand derart am harnleitenden Gefäß angeordnet ist, dass bei einem Befüllen der hydraulischen Okklusionsvorrichtung mit einem Hydraulikfluid ein Okkludieren des harnleitenden Gefäßes erfolgt. Dieses kann somit durch die Okklusionsvorrichtung verschlossen werden. Zum Freigeben des harnleitenden Gefäßes ist die hydraulische Okklusionsvorrichtung wieder entleerbar.

Der künstliche Sphinkter weist ein implantierbares elastisches Reservoir auf, das fluidisch mit der Okklusionsvorrichtung verbunden ist, sodass das Hydraulikfluid vom elastischen Reservoir in Richtung der Okklusionsvorrichtung und zurück strömen können. Hierbei wird die Strömung des Hydraulikfluids vom elastischen Reservoir zur hydraulischen Okklusionsvorrichtung, insbesondere ausschließlich, durch die Eigenelastizität des elastischen Reservoirs verursacht. Es sind somit keine weiteren Komponenten, insbesondere Pumpen, vorgesehen, durch die das Hydraulikfluid vom elastischen Reservoir in Richtung der hydraulischen Okklusionsvorrichtung gefördert wird. Sofern somit eine fluidische Verbindung zwischen dem elastischen Reservoir und der Okklusionsvorrichtung besteht, ist die Okklusionsvorrichtung derart mit Hydraulikfluid befüllt, dass sie das harnleitende Gefäß verschließt.

Damit der Patient seine Blase entleeren kann, weist der erfindungsgemäße künstliche Sphinkter eine erste elektrisch angetriebene Pumpe zum Pumpen des Hydraulikfluids gegen die durch die Eigenelastizität des elastischen Reservoirs verursachte Kraft von der hydraulischen Okklusionsvorrichtung zum elastischen Reservoir auf. Diese erste elektrisch angetriebene Pumpe wird vom Patienten im Normalbetrieb aktuiert, damit der Patient seine Blase entleeren kann. Das Aktuieren kann beispielsweise über eine außerhalb des Körpers angeordnete Fernbedienung oder über einen Knopf erfolgen, den der Patient an der Pumpe betätigen kann. Auch kann innerhalb des Körpers des Patienten eine Fernbedienung vorgesehen sein, z.B. indem ein Betätigungsknopf subkutan angeordnet ist und der somit durch den Patienten einfach betätigt werden kann. Das Betätigen der elektrisch angetriebenen Pumpe ist mit keiner großen Anstrengung für den Patienten verbunden, da er nicht durch eigene Kraft das Hydraulikfluid von der Okklusionsvorrichtung gegen die Eigenelastizität des elastischen Reservoirs pumpen muss. Der erfindungsgemäße künstliche Sphinkter eignet sich somit in besonderer Weise für körperlich schwache und ältere Patienten. Im Normalbetrieb, d.h. wenn die erste elektrische Pumpe funktionsfähig ist, sind somit keine feinmotorischen Fertigkeiten bei dem Patienten notwendig, um die Pumpe zu bedienen. Bei Okklusionsvorrichtungen, die aus dem Stand der Technik bekannt sind, wird dagegen bspw. im Skrotum des Patienten eine mechanische Pumpe implantiert, die kompliziert zu bedienen ist. Erfindungsgemäß ist auch eine Anwendung bei gelähmten Patienten möglich, da die Pumpe durch die Fernbedienung von anderen Personen bedient werden kann.

Bevorzugt weist der künstliche Sphinkter ferner eine zweite Notfallpumpe auf, durch die ebenfalls das Hydraulikfluid von der hydraulischen Okklusionsvorrichtung in Richtung des elastischen Reservoirs förderbar ist. Diese zweite Notfallpumpe ist für den Notfallbetrieb vorgesehen, nämlich bei einer nicht ordnungsgemäßen Funktion der ersten Pumpe. Sollte beispielsweise die erste elektrische Pumpe ausfallen, kann der Patient immer noch durch Betätigen der zweiten Notfallpumpe seine Blase entleeren.

Die zweite Notfallpumpe ist bevorzugt parallel zur ersten elektrischen Pumpe zwischen dem elastischen Reservoir und der hydraulischen Okklusionsvorrichtung angeordnet. Bei der Notfallpumpe kann es sich um eine mechanische oder elektrische Pumpe handeln. Das Verwenden einer mechanischen Pumpe als Notfallpumpe ist bevorzugt, da die Notfallpumpe ohnehin sehr selten eingesetzt wird.

Der erfindungsgemäße Sphinkter weist den Vorteil auf, dass der maximale Druck in der hydraulischen Okklusionsvorrichtung nicht immer identisch mit dem Druck im elastischen Reservoir sein muss. Somit ist es möglich, im elastischen Reservoir einen relativ hohen Druck vorzusehen, der der hydraulischen Okklusionsvorrichtung in Stresssituationen zugeführt werden kann, um auch in diesen Situationen das harnleitende Gefäß sicher zu verschließen. Im Standardbetrieb dagegen, d. h. wenn keine Stresssituation vorliegt, kann der hydraulischen Okklusionsvorrichtung ein geringerer Druck zugeführt werden, der jedoch ausreichend ist, um das harnleitende Gefäß während des Standardbetriebs sicher zu verschließen. Zum Erreichen dieses Standarddrucks während des Standardbetriebs, kann beispielswiese ein Teil des Hydraulikfluids aus der hydraulischen Okklusionsvorrichtung durch die erste elektrische Pumpe wieder zurück in das elastische Reservoir gefördert werden. In Abhängigkeit der Menge des Hydraulikfluids, das zurück in das elastische Reservoir gefördert wird, kann der Druck in der hydraulischen Okklusionsvorrichtung zwischen dem Maximaldruck, der in dem elastischen Reservoir herrscht, und einem drucklosen Zustand (d. h. demjenigen Zustand, in dem die Okklusionsvorrichtung vollständig entleert ist) angepasst werden.

Das zu verschließende harnleitende Gefäß wird somit nicht kontinuierlich mit dem hohen Druck verschlossen, der notwendig ist, um einen sicheren Verschluss während einer Stresssituation zu gewährleisten. Hierdurch kann eine Schädigung des harnleitenden Gefäßes vermieden werden.

Es wird bevorzugt, dass die erste elektrische Pumpe eine monodirektionale Pumpe ist, durch die das Hydraulikfluid ausschließlich von der hydraulischen Okklusionsvorrichtung in Richtung des elastischen Reservoirs und nicht in die entgegengesetzte Richtung förderbar ist. Das Verwenden einer monodirektionalen Pumpe ist ausreichend, da das Fördern des Hydraulikfluids vom elastischen Reservoir in Richtung der hydraulischen Okklusionsvorrichtung vorzugsweise ausschließlich durch die Eigenelastizität des elastischen Reservoirs erfolgt.

In einer bevorzugten Ausführungsform weist der erfindungsgemäße Sphinkter ein steuerbares Ventil auf, das an einer Hydraulikleitung, insbesondere parallel zu ersten Pumpe, angeordnet ist. Dieses steuerbare Ventil dient dem Verschließen und Freigeben dieser Hydraulikleitung in Reaktion auf eine Ansteuerung des steuerbaren Ventils durch eine Steuervorrichtung. Die durch das steuerbare Ventil verschließbare Hydraulikleitung verläuft zwischen dem elastischen Reservoir und der hydraulischen Okklusionsvorrichtung. Durch das genannte steuerbare Ventil kann somit die Hydraulikleitung zwischen dem elastischen Reservoir und der Okklusionsvorrichtung wahlweise geöffnet oder verschlossen werden. In Abhängigkeit von der Betätigung des steuerbaren Ventils kann somit gesteuert werden, wie viel Hydraulikfluid der Okklusionsvorrichtung aus dem Reservoir zugeführt wird. Ein frühes Verschließen des steuerbaren Ventils während des Befüllvorgangs der Okklusionsvorrichtung wird beispielsweise dazu führen, dass diese nicht mit einem hohen Druck beaufschlagt wird. Ein sehr spätes Verschließen oder gar kein Verschließen des steuerbaren Ventils wird dagegen dazu führen, dass die Okklusionsvorrichtung mit einem hohen Druck beaufschlagt wird, der unter Umständen sogar dem Maximaldruck, der im Reservoir herrscht, entsprechen kann.

Der erfindungsgemäße Sphinkter weist ferner bevorzugt einen Sensor zum direkten und/oder indirekten Erfassen eines Drucks und/oder Druckanstiegs im harnleitenden Gefäß auf. Auf Basis der Signale des Sensors erfolgt ein Ansteuern des steuerbaren Ventils und/oder der ersten Pumpe. Wird beispielsweise ein plötzlicher Druckanstieg im harnleitenden Gefäß durch den Sensor detektiert, so kann von einer Stresssituation ausgegangen werden. Beispielsweise ist es möglich, dass der Patient gerade hustet. In Reaktion auf diesen erfassten plötzlichen Druckanstieg kann durch die Steuervorrichtung das steuerbare Ventil geöffnet werden, sodass das im elastischen Reservoir befindliche Hydraulikfluid ruckartig in die Okklusionsvorrichtung strömt. Es erfolgt somit eine sehr schnelle Erhöhung des in der Okklusionsvorrichtung vorherrschenden Drucks, sodass das harnleitende Gefäß auch in der vorliegenden Stresssituation sicher verschlossen werden kann. Bei dem Sensor kann es sich um einen separaten Sensor handeln, der bspw. am harnleitenden Gefäß, an der Blase oder auch an beliebiger Stelle im Peritoneum/Abdomen angeordnet ist. Alternativ oder zusätzlich ist es möglich, eine Druckänderung direkt an der hydraulischen Okklusionsvorrichtung zu detektieren. Dies ist möglich, da ein schneller Druckanstieg im harnleitenden Gefäß auch zu einer entsprechenden Druckänderung in der Okklusionsvorrichtung führt. Hierdurch kann auf einen zusätzlichen Sensor verzichtet werden. Bei dieser Ausführungsform wird daher die hydraulische Okklusionsvorrichtung selbst als Sensor verwendet.

Unabhängig von der Art des verwendeten Sensors, ist es möglich den absoluten Druck im harnleitenden Gefäß und/oder einen Druckanstieg, d.h. die Veränderung des Drucks über die Zeit zu messen. Sofern ausschließlich ein Druckanstieg gemessen wird, ist es nicht notwendig den absoluten Druck zu ermitteln. Ein ausschließliches Erkennen eines Druckanstiegs, der einen bestimmten Grenzwert überschreitet, ist ausreichend, um eine Stresssituation zu erkennen und basierend hierauf den Druck in der hydraulischen Okklusionsvorrichtung auf den Stressdruck zu erhöhen.

Eine Stresssituation kann bspw. angenommen werden, wenn der Abdominaldruck eine Druckanstiegsrate von mehr als 36 mmHg/s, bevorzugt 70 mmHg/s und besonders bevorzugt 110 mmHg/s aufweist. Die Druckanstiegsrate kann bspw. mittels einer CLP-Messung durchgeführt werden. Erläuterungen hierzu sind in folgender Veröffentlichung zu finden:
[1] Palmtag, Goepel, Heidler: Urodynamik 2. Auflage, S. 109 http://www.springerlink.com/content/978-3-540-72505-3/#section=319686&page=1

Die dargestellten Druckänderungsraten können auch bei einer Druckmessung in anderen Bereichen, bspw. an der Blase oder über den Druck der hydraulischen Okklusionsvorrichtung angewendet werden.

Ferner kann eine Stresssituation angenommen werden, wenn ein Abdominaldruck von 55 mmHG gemessen wird. Eine Stresssituation kann angenommen werden, wenn eins der beiden Kriterien, nämlich eine erhöhte Druckänderungsrate oder ein erhöhter absoluter Abdominaldruck detektiert wird.

Eine Detektion einer Stresssituation durch die genannten Grenzdrücke ist sinnvoll, wenn der erfindungsgemäße Sphinkter nicht dynamisch auf jegliche Druckänderung reagiert und in diesem Fall der in der hydraulischen Okklusionsvorrichtung anliegende Druck zu jedem Zeitpunkt an dem vorherrschenden Druck im biologischen System angepasst wird. In dieser Ausführungsform ist der Druck in der hydraulischen Okklusionsvorrichtung auch in einer Stresssituation immer nur exakt so groß wie gerade notwendig. Hierbei handelt es somit um eine kontinuierliche Druckregelung. Alternativ ist es wie oben dargestellt möglich, durch die genannten Kriterien eine Stresssituation zu detektieren und sofern eine solche detektiert wurde, unabhängig vom tatsächlichen Stressdruck im biologischen System, den Druck in der hydraulischen Okklusionsvorrichtung auf einen definierten Stressdruck zu erhöhen. Bevorzugt existieren somit in dieser Ausführungsform für den Druck in der hydraulischen Okklusionsvorrichtung lediglich zwei Zustände, nämlich einer für die Normalsituation (Normaldruck) und einer für jegliche Stresssituation (Stressdruck), der unabhängig vom tatsächlichen Stressdruck im biologischen System ist. Dies hat den Vorteil, dass ein deutlich geringerer Regelaufwand besteht und die Druckmessung im biologischen System vereinfacht werden kann.

Es ist bevorzugt, dass die zweite Notfallpumpe, einen Deaktivierungsmechanismus aufweist, sodass die Hydraulikleitung, in der sich diese Notfallpumpe befindet, im Normalbetrieb gesperrt ist. Dies bedeutet, dass durch diese Hydraulikleitung kein Hydraulikfluid strömen kann, sodass das Hydraulikfluid im Normalbetrieb lediglich durch die erste elektrische Pumpe und ggf. durch das steuerbare Ventil strömt.

Weiterhin ist es bevorzugt, dass die erste elektrische Pumpe und das steuerbare Ventil jeweils einen Deaktivierungsmechanismus aufweisen, sodass die beiden Hydraulikleitungen, in denen sich die erste elektrische Pumpe und das steuerbare Ventil befinden, im Notfall betrieb gesperrt werden können, sodass dann das Hydraulikfluid durch die zweite Notfallpumpe von der hydraulischen Okklusionsvorrichtung zum Reservoir gepumpt werden kann, ohne dass es über das steuerbare Ventil oder die erste elektrische Pumpe wieder zur Okklusionsvorrichtung zurückströmt.

Es ist bevorzugt, dass das Durchlassvolumen des steuerbaren Ventils einstellbar ist, indem z. B. das steuerbare Ventil mehr oder weniger geöffnet wird. Dies kann in Abhängigkeit der vom Sensor erfassten Signale erfolgen.

Es ist weiterhin bevorzugt, dass die Steuervorrichtung ausgebildet ist zum Ausgeben eines Steuerbefehls zum vollständigen Öffnen des steuerbaren Ventils, wenn der durch den Sensor erfasste Druck im harnleitenden Gefäß einen definierten Grenzdruck überschreitet. Hierdurch erfolgt ein durch die Eigenelastizität des elastischen Behälters verursachtes ruckartiges Befüllen der hydraulischen Okklusionsvorrichtung mit Hydraulikfluid, bis in der Okklusionsvorrichtung und dem Reservoir das gleiche Druckniveau erreicht ist oder bis das steuerbare Ventil wieder verschlossen wird. Durch das Zusammenspiel des steuerbaren Ventils und des elastischen Reservoirs kann in einer Stresssituation ein sehr viel schnelleres Befüllen der hydraulischen Okklusionsvorrichtung mit Hydraulikfluid erreicht werden, als wenn eine elektrische oder manuelle Pumpe zum Fördern des Hydraulikfluids in die Okklusionsvorrichtung verwendet wird. Beim Verwenden einer Pumpe kann nicht sichergestellt werden, dass der Druckaufbau in der Okklusionsvorrichtung ausreichend schnell erfolgt, sodass ein sicherer Verschluss des harnleitenden Gefäßes in einer Stresssituation nicht immer erreicht wird.

Durch das vollständige Öffnen des steuerbaren Ventils in einer Stresssituation kann es vorkommen, dass der Druck in der hydraulischen Okklusionsvorrichtung höher wird als nötig. Dies kann dadurch geschehen, dass durch ein vollständiges Öffnen des steuerbaren Ventils ein sehr schneller Druckanstieg in der hydraulischen Okklusionsvorrichtung erfolgt. Somit ist der Druck in der Okklusionsvorrichtung möglicherweise auch höher als es nötig wäre, um in der vorliegenden Stresssituation das harnleitende Gefäß sicher zu verschließen. Dies führt jedoch zu keiner dauerhaften Schädigung des harnleitenden Gefäßes, da durch die erste elektrische Pumpe kurze Zeit nach dem ruckartigen Befüllen der Okklusionsvorrichtung diese wieder zum Teil entleert werden kann. Dies kann auch stufenweise geschehen, sodass ein erstes Entleeren bis zu dem Punkt erfolgt, an dem die Okklusionsvorrichtung den Druck erreicht hat, der notwendig ist, um in der vorliegenden Stresssituation das harnleitende Gefäß sicher zu verschließen. In einem weiteren Schritt, der beispielsweise in einigen Minuten folgen kann, ist es möglich, weiteres Hydraulikfluid durch die elektrische Pumpe aus der Okklusionsvorrichtung heraus zu fördern, sodass wieder der Standarddruck erreicht wird, der ausreichend ist, um im Standardbetrieb das harnleitende Gefäß sicher zu verschließen.

Der erfindungsgemäße Sphinkter ermöglicht es ferner, sehr flexibel auf individuelle Anforderungen des Patienten zu reagieren. Beispielsweise kann es nach der Implantation des Systems zu anatomischen Veränderungen im Körper des Patienten kommen, die es notwendig machen, das harnleitende Gefäß mit einem höheren oder niedrigen Druck als ursprünglich vorgesehen zu verschließen. Hierauf kann sehr einfach durch eine Veränderung der Ansteuerung des steuerbaren Ventils und/oder der elektrischen Pumpe reagiert werden.

Die Erfindung betrifft ferner ein Verfahren zum Steuern eines künstlichen Sphinkters zum steuerbaren Okkludieren eines harnleitenden Gefäßes bei einem inkontinenten Patienten. Das Verfahren eignet sich insbesondere zum Steuern eines künstlichen Sphinkters, wie er in der vorliegenden Anmeldung beschrieben ist. Das erfindungsgemäße Verfahren weist die folgenden Schritte auf:
Zunächst erfolgt ein direktes oder indirektes Erfassen eines Drucks und/oder Druckanstiegs im harnleitenden Gefäß durch einen Sensor. Dies kann beispielsweise dadurch erfolgen, dass direkt am harnleitenden Gefäß ein Drucksensor oder ein Dehnungssensor angeordnet ist. Alternativ oder zusätzlich kann ein Druckanstieg in der Blase des Patienten erfasst werden. Dies kann beispielsweise ebenfalls durch Verwendung eines Druck- oder Dehnungssensors erfolgen. Bei einer Druckmessung in der Blase ist es auch möglich, den Blasenfüllstand zu ermitteln. Alternativ oder zusätzlich kann auch eine Druckmessung durch Druck- oder Dehnungssensoren an beliebiger Stelle im Peritoneum oder im Abdomen erfolgen.

Alternativ zu einer direkten Erfassung des Drucks und/oder Druckanstiegs durch Verwendung eines Sensors, ist es auch möglich, eine Druckänderung an der Okklusionsvorrichtung selbst zu detektieren. Wenn der Druck im harnleitenden Gefäß ansteigt, ist diese Druckänderung auch an der Okklusionsvorrichtung messbar. Beispielsweise ist es möglich, einen starken Druckanstieg an der Okklusionsvorrichtung zu messen. Hierdurch können Stresssituationen erkannt werden. Ggf. ist es auch möglich, den Blasenfüllstand zu erkennen.

Es erfolgt ein Öffnen eines steuerbaren Ventils, das in einer Fluidleitung zwischen einem elastischen Reservoir und einer hydraulischen Okklusionsvorrichtung angeordnet ist. Hierbei kann es sich auch um eine sehr kurze Fluidleitung handeln. Das Öffnen des steuerbaren Ventils erfolgt hierbei, wenn der durch den Sensor gemessene Druck einen definierten Grenzdruck überschreitet. Beim Öffnen des Ventils erfolgt durch die Eigenelastizität des elastischen Reservoirs ein Strömen des Hydraulikfluids vom elastischen Reservoir zur hydraulischen Okklusionsvorrichtung, bis in der Okklusionsvorrichtung und dem Reservoir das gleiche Druckniveau erreicht wird oder das steuerbare Ventil wieder verschlossen wird.

Das erfindungsgemäße Verfahren kann sämtliche Merkmale aufweisen, die in Zusammenhang mit der erfindungsgemäßen Vorrichtung beschrieben wurden, und umgekehrt.

Es ist bevorzugt, dass das steuerbare Ventil vollständig geöffnet wird, wenn der Druck, der durch den Sensor erfasst wird, den Grenzdruck überschreitet. Hierdurch erfolgt ein ruckartiges Befüllen der hydraulischen Okklusionsvorrichtung mit Hydraulikfluid bis zu einem Stressdruck, der ausreichend ist, um das harnleitende Gefäß während einer Stresssituation sicher zu verschließen.

Weiterhin ist es bevorzugt, dass nach dem ruckartigen Befüllen der hydraulischen Okklusionsvorrichtung ein Teil des Hydraulikfluids durch eine insbesondere elektrisch angetriebene Pumpvorrichtung wieder zurück in das elastische Reservoir befördert wird.

Weiterhin ist es bevorzugt, dass der Druck in der hydraulischen Okklusionsvorrichtung auf einen Standarddruck reduziert wird, der ausreichend ist, um das harnleitende Gefäß im Standardbetrieb sicher zu verschließen. Eine derartige Druckreduktion erfolgt nach einer Stresssituation, d. h .wenn die Stresssituation nicht mehr vorliegt.

Es ist möglich, sowohl den Standarddruck als auch den Stressdruck vom gemessenen bzw. abgeleiteten Druck im harnleitenden Gefäß abhängig zu machen. So ist es bspw. möglich, dass der Standarddruck nicht immer gleich ist, sondern ebenfalls an verschiedene Situationen angepasst wird. Alternativ ist es möglich, lediglich Stresssituationen zu erfassen, indem bspw. ein schneller Druckanstieg im harnleitenden Gefäß detektiert wird und einen Standarddruck zum Verschließen des harnleitenden Gefäßes außerhalb von Stresssituationen festzulegen. Dieser wird dann im Normalbetrieb nicht geändert, könnte aber durch Neuprogrammierung des erfindungsgemäßen Sphinkters geändert werden.

Im Folgenden wird eine bevorzugte Ausführungsform der Erfindung anhand einer Figur erläutert.

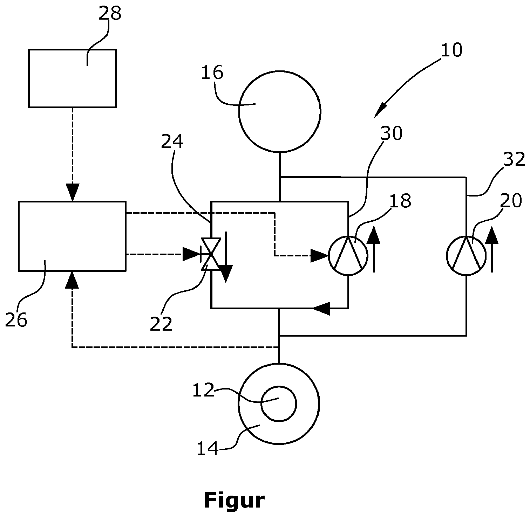

Die Figur zeigt eine schematische Darstellung des erfindungsgemäßen künstlichen Sphinkters.

Der künstliche Sphinkter 10 weist eine hydraulische Okklusionsvorrichtung 14 auf, die ringförmig und vorzugsweise torusförmig ausgebildet ist und das harnleitende Gefäß 12 umschließt. Der künstliche Sphinkter 10 umfasst ferner ein elastisches Reservoir 16, das über eine Hydraulikleitung 24 mit der hydraulischen Okklusionsvorrichtung 14 verbunden ist. In dieser Hydraulikleitung 24 befindet sich das steuerbare Ventil 22, das durch die Steuervorrichtung 26 gesteuert wird. Dies erfolgt in Reaktion auf die durch den Sensor 28 erfassten Signale. Der Sensor 28 misst insbesondere den Druck im harnleitenden Gefäß 12. Die Steuervorrichtung 26 steuert ferner die elektrische Pumpe 18 an, die in einer parallelen Hydraulikleitung 30 angeordnet ist. Diese Hydraulikleitung 30 verläuft ebenfalls vom elastischen Reservoir 16 zur hydraulischen Okklusionsvorrichtung 14 und zwar in einer Richtung parallel zur Hydraulikleitung 24.

Weiterhin parallel zu diesen beiden Leitungen verläuft eine weitere Hydraulikleitung 32, in der die zweite Notfallpumpe 20 angeordnet ist. Durch diese Pumpe kann bei einem nicht ordnungsgemäßen Betrieb der ersten elektrischen Pumpe 18 das Hydraulikfluid von der Okklusionsvorrichtung 14 in Richtung des elastischen Reservoirs 16 gefördert werden.

Das Durchlassvolumen des steuerbaren Ventils 22 ist durch die Steuervorrichtung 26 einstellbar. So ist es beispielsweise möglich, das Steuerventil 22 nur zum Teil zu öffnen, wenn der Druck in der Okklusionsvorrichtung 14 langsam ansteigen soll. Dies kann beispielsweise hilfreich sein, wenn der Verschlussdruck in der Okklusionsvorrichtung 14 in Abhängigkeit vom Blasenfüllstand eingestellt werden soll.

Die Energieversorgung des Gesamtsystems 10 kann durch einen nicht dargestellten Akku erfolgen. Auch ist eine induktive drahtlose Energieübertragung möglich, die aus dem Stand der Technik bekannt ist.

## Patentansprüche

1. Künstlicher Sphinkter zum steuerbaren Okkludieren eines harnleitenden Gefäßes (12) bei einem inkontinenten Patienten, mit
einer implantierbaren, insbesondere ringförmigen hydraulischen Okklusionsvorrichtung (14), die in implantiertem Zustand derart am harnleitenden Gefäß (12) angeordnet ist, dass bei einem Befüllen der hydraulischen Okklusionsvorrichtung (14) mit einem Hydraulikfluid ein Okkludieren des harnleitenden Gefäßes (12) erfolgt und die hydraulische Okklusionsvorrichtung (14) zum Freigeben des harnleitenden Gefäßes (12) wieder entleerbar ist,
einem implantierbaren elastischen Reservoir (16), das fluidisch mit der Okklusionsvorrichtung (14) verbunden ist, sodass das Hydraulikfluid vom elastischen Reservoir (16) in Richtung der hydraulischen Okklusionsvorrichtung (14) und zurück strömen kann, wobei die Strömung des Hydraulikfluids vom elastischen Reservoir (16) zur hydraulischen Okklusionsvorrichtung (14) durch die Eigenelastizität des elastischen Reservoirs (16) erfolgt,
einer ersten elektrisch angetriebene Pumpe (18) zum Pumpen des Hydraulikfluids gegen die durch die Eigenelastizität des Reservoirs (16) verursachte Kraft von der hydraulischen Okklusionsvorrichtung (14) zum elastischen Reservoir (16) im Normalbetrieb,
**gekennzeichnet durch** ein steuerbares Ventil (22), das an einer Hydraulikleitung (24) zwischen dem elastischen Reservoir (16) und der hydraulischen Okklusionsvorrichtung (14), insbesondere parallel zur ersten Pumpe (18), angeordnet ist, zum Verschließen und Freigeben dieser Hydraulikleitung (24) in Reaktion auf eine Ansteuerung des steuerbaren Ventils (22) **durch** eine Steuervorrichtung (26),
einen Sensor (28) zum direkten und/oder indirekten Erfassen eines Drucks und/oder Druckanstiegs im harnleitenden Gefäß (12), wobei auf Basis der Signale dieses Sensors (28) ein Ansteuern des steuerbaren Ventils (22) und/oder der Pumpe (18) erfolgt,
wobei die Steuervorrichtung (26) zum Ausgeben eines Steuerbefehls zum vollständigen Öffnen des steuerbaren Ventils (22) ausgebildet ist, wenn der **durch** den Sensor (28) erfasste Druck im harnleitenden Gefäß (12) einen definierten Grenzdruck überschreitet, sodass **durch** die Eigenelastizität des elastischen Behälters (16) ein ruckartiges Befüllen der hydraulischen Okklusionsvorrichtung (14) mit Hydraulikfluid erfolgt, bis in der Okklusionsvorrichtung (14) und dem Reservoir (16) das gleiche Druckniveau erreicht ist oder das steuerbare Ventil (22) wieder verschlossen wird.

2. Künstlicher Sphinkter nach Anspruch 1 **dadurch gekennzeichnet, dass** die erste Pumpe (18) eine monodirektionale Pumpe ist, durch die das Hydraulikfluid ausschließlich von der hydraulischen Okklusionsvorrichtung (14) in Richtung des elastischen Reservoirs (16) und nicht in die entgegengesetzte Richtung förderbar ist.

3. Künstlicher Sphinkter nach Anspruch 1 oder 2, **dadurch gekennzeichnet, dass** das Durchlassvolumen des steuerbaren Ventils (22) einstellbar ist, insbesondere in Abhängigkeit von den vom Sensor (28) erfassten Signalen.

4. Künstlicher Sphinkter nach einem der Ansprüche 1 - 3, **gekennzeichnet durch** eine zweite Notfallpumpe (20) zum Pumpen des Hydraulikfluids gegen die **durch** die Eigenelastizität des Reservoirs (16) verursachte Kraft von der hydraulischen Okklusionsvorrichtung (14) zum elastischen Reservoir (16) im Notfallbetrieb, nämlich bei einer nicht ordnungsgemäßen Funktion der ersten Pumpe (18).

5. Verfahren zum Steuern eines künstlichen Sphinkters (10) zum steuerbaren Okkludieren eines harnleitenden Gefäßes (12) bei einem inkontinenten Patient, insbesondere zum Betreiben eines künstlichen Sphinkters nach einem der Ansprüche 1 - 3, mit den Schritten:
direktes oder indirektes Erfassen eines Drucks und/oder Druckanstiegs in einem harnleitenden Gefäß (12) durch einen Sensor (28),
Öffnen eines steuerbaren Ventils (22), das in einer Fluidleitung (24) zwischen einem elastischen Reservoir (16) und einer hydraulischen Okklusionsvorrichtung (14) angeordnet ist, wobei das Öffnen erfolgt, wenn der durch den Sensor (28) gemessene Druck einen definierten Grenzdruck überschreitet,
wobei beim Öffnen des Ventils (22) durch die Eigenelastizität des elastischen Reservoirs (16) ein Strömen des Hydraulikfluids vom elastischen Reservoir (16) zur hydraulischen Okklusionsvorrichtung (14) erfolgt, bis in der Okklusionsvorrichtung (14) und dem Reservoir (16) das gleiche Druckniveau erreicht ist oder das steuerbare Ventil (22) wieder verschlossen wird,
**dadurch gekennzeichnet, dass** das steuerbare Ventil (22) vollständig geöffnet wird, wenn der Druck, der durch den Sensor (28) gemessen wird, den Grenzdruck überschreitet, sodass ein ruckartiges Befüllen der hydraulischen Okklusionsvorrichtung (14) mit Hydraulikfluid bis zu einem Stressdruck erfolgt, der ausreichend ist, um das harnleitende Gefäß (12) während einer Stresssituation sicher zu verschließen.

6. Verfahren nach Anspruch 5 **dadurch gekennzeichnet, dass** nach dem ruckartigen Befüllen der hydraulischen Okklusionsvorrichtung (14) ein Teil des Hydraulikfluids durch eine insbesondere elektrisch angetriebene Pumpvorrichtung (18) wieder zurück in das elastische Reservoir (16) gefördert wird.

7. Verfahren nach Anspruch 6 **dadurch gekennzeichnet, dass** der Druck in der hydraulischen Okklusionsvorrichtung auf einen Standarddruck reduziert wird, der ausreichend ist, um das harnleitende Gefäß (12) im Standardbetrieb sicher zu verschließen.

## Claims

1. An artificial sphincter for controllably occluding a urine-carrying vessel (12) of an incontinent patient, comprising
an implantable, in particular annular hydraulic occlusion device (14) which, when implanted, is arranged at said urine-carrying vessel (12) in such a manner that, during filling of said hydraulic occlusion device (14) with a hydraulic fluid, said urine-carrying vessel (12) is occluded and said hydraulic occlusion device (14) is adapted to be emptied again for clearing said urine-carrying vessel (12),
an implantable elastic reservoir (16) which is in fluid communication with said occlusion device (14) such that the hydraulic fluid can flow from said elastic reservoir (16) towards said hydraulic occlusion device (14) and back, wherein the flow of the hydraulic fluid from said elastic reservoir (16) to said hydraulic occlusion device (14) is caused by the inherent elasticity of said elastic reservoir (16),
a first electrically driven pump (18) for pumping the hydraulic fluid against the force produced by the inherent elasticity of said reservoir (16) from said hydraulic occlusion device (14) to said elastic reservoir (16) during normal operation,
**characterized by** a controllable valve (22) which is arranged, in particular in parallel to the first pump (18), at a hydraulic line (24) between the elastic reservoir (16) and the hydraulic occlusion device (14) for closing and clearing said hydraulic line (24) in response to control of said controllable valve (22) by a control device (26),
a sensor (28) for directly and/or indirectly sensing a pressure and/or pressure increase in the urine-carrying vessel (12), wherein, on the basis of the signals from said sensor (28), control of the controllable valve (22) and/or the pump (18) is performed,
the control device (26) being configured to output a control command for completely opening the controllable valve (22) when the pressure in the urine-carrying vessel (12) sensed by the sensor (28) exceeds a defined limit pressure such that, due to the inherent elasticity of the elastic reservoir (16), a jerky filling of the hydraulic occlusion device (14) with hydraulic fluid is performed until said occlusion device (14) and said reservoir (16) have reached the same pressure level or said controllable valve (22) is closed again.

2. The artificial sphincter according to claim 1, **characterized in that** the first pump (18) is a monodirectional pump through which the hydraulic fluid is adapted to be exclusively delivered from the hydraulic occlusion device (14) towards the elastic reservoir (16) and not in the opposite direction.

3. The artificial sphincter according to claim 1 or 2, **characterized in that** the passage volume of the controllable valve (22) is adapted to be adjusted in particular as a function of the signals sensed by the sensor (28).

4. The artificial sphincter according to any one of claims 1 - 3, **characterized by** a second emergency pump (20) for pumping the hydraulic fluid against the force produced by the inherent elasticity of said reservoir (16) from said hydraulic occlusion device (14) to said elastic reservoir (16) during emergency operation, namely when said first pump (18) does not function properly.

5. A method for controlling an artificial sphincter (10) for controllably occluding a urine-carrying vessel (12) of an incontinent patient, in particular for operating an artificial sphincter according to any one of claims 1 - 3, comprising the steps of:
directly or indirectly sensing a pressure and/or pressure increase in a urine-carrying vessel (12) by a sensor (28),
opening a controllable valve (22) which is arranged in a fluid line (24) between an elastic reservoir (16) and a hydraulic occlusion device (14), wherein opening is performed when the pressure measured by said sensor (28) exceeds a defined limit pressure,
wherein, during opening of said valve (22), the inherent elasticity of said elastic reservoir (16) causes the hydraulic fluid to flow from said elastic reservoir (16) to said hydraulic occlusion device (14) until said occlusion device (14) and said reservoir (16) have reached the same pressure level or said controllable valve (22) is closed again,
**characterized in that** the controllable valve (22) is completely opened when the pressure measured by the sensor (28) exceeds the limit pressure such that a jerky filling of the hydraulic occlusion device (14) with hydraulic fluid up to a stress pressure is performed which is sufficient to reliably close the urine-carrying vessel (12) during a stress situation.

6. The method according to claim 5, **characterized in that**, after the jerky filling of the hydraulic occlusion device (14), part of the hydraulic fluid is delivered back again into the elastic reservoir (16) by a pumping device (18) in particular by of electrically driven type.

7. The method according to claim 6, **characterized in that** the pressure in the hydraulic occlusion device is reduced to a standard pressure which is sufficient to reliably close the urine-carrying vessel (12) during standard operation.

## Revendications

1. Sphincter artificiel destiné à l'occlusion contrôlable d'un vaisseau (12) conduisant l'urée chez un patient incontinent, avec :
un dispositif d'occlusion (14) implantable, en particulier hydraulique et de forme circulaire qui, à l'état implanté, est disposé de telle sorte au niveau du vaisseau (12) conduisant l'urée qu'une occlusion du vaisseau (12) conduisant l'urée se produit quand le dispositif d'occlusion (14) hydraulique est rempli avec un fluide hydraulique et le dispositif d'occlusion (14) hydraulique peut être de nouveau purgé en vue de l'ouverture du vaisseau (12) conduisant l'urée ;
un réservoir (16) élastique implantable qui est relié de manière fluidique au dispositif d'occlusion (14), de telle sorte que le fluide hydraulique peut aussi bien circuler depuis le réservoir (16) élastique dans la direction du dispositif d'occlusion (14) hydraulique que dans la direction inverse, où le courant du fluide hydraulique se produit depuis le réservoir (16) élastique jusqu'au dispositif d'occlusion (14) hydraulique du fait de la propre élasticité du réservoir (16) élastique ;
une première pompe (18) à commande électrique destinée au pompage du fluide hydraulique à l'encontre de la force provoquée par la propre élasticité du réservoir (16), depuis le dispositif d'occlusion (14) hydraulique jusqu'au réservoir (16) élastique, dans le mode de fonctionnement normal,
**caractérisé par** une valve (22) contrôlable, laquelle est disposée au niveau d'une conduite hydraulique (24) se trouvant entre le réservoir (16) élastique et le dispositif d'occlusion (14) hydraulique, en particulier parallèlement à la première pompe (18), en vue de la fermeture et de l'ouverture de cette conduite hydraulique (24) en réaction à une commande de la valve (22) contrôlable, par l'intermédiaire d'un dispositif de commande (26), ainsi que
par une sonde (28) en vue de la détection directe et/ou indirecte d'une pression et/ou d'une croissance de la pression dans le vaisseau (12) conduisant l'urée, où un pilotage de la valve (22) contrôlable et/ou de la pompe (18) se produit sur la base des signaux émis par cette sonde (28),
où le dispositif de commande (26) destiné à la transmission d'une instruction de commande en vue de l'ouverture complète de la valve (22) contrôlable est conçu de telle sorte que, quand la pression saisie par la sonde (28) dans le vaisseau (12) conduisant l'urée dépasse une pression limite définie, un remplissage du dispositif d'occlusion (14) hydraulique avec le fluide hydraulique a lieu par à-coup du fait de la propre élasticité du réservoir (16) élastique, le remplissage se produit jusqu'à ce que le même niveau de pression soit atteint, tant dans le dispositif d'occlusion (14) que dans le réservoir (16), ou jusqu'à ce que la valve (22) contrôlable soit de nouveau fermée.

2. Sphincter artificiel selon la revendication 1 **caractérisé en ce que** la première pompe (18) est une pompe unidirectionnelle, par laquelle le fluide hydraulique peut être transporté exclusivement depuis le dispositif d'occlusion (14) hydraulique dans la direction du réservoir (16) élastique et pas dans la direction opposée.

3. Sphincter artificiel selon la revendication 1 ou 2, **caractérisé en ce que** le débit de la valve (22) contrôlable est réglable, en particulier en fonction des signaux captés par la sonde (28).

4. Sphincter artificiel selon l'une des revendications 1 à 3, **caractérisé par** une deuxième pompe d'urgence (20) destinée au pompage du fluide hydraulique à l'encontre de la force provoquée par la propre élasticité du réservoir (16), depuis le dispositif d'occlusion (14) hydraulique jusqu'au réservoir (16) élastique en mode de secours, notamment quand la première pompe (18) ne fonctionne pas correctement.

5. Procédé destiné à la commande d'un sphincter (10) artificiel en vue de l'occlusion contrôlable d'un vaisseau (12) conduisant l'urée chez un patient incontinent, en particulier en vue du fonctionnement d'un sphincter artificiel selon l'une des revendications 1 à 3, ledit procédé comprenant les étapes suivantes :
la détection directe et/ou indirecte d'une pression et/ou d'une croissance de la pression dans un vaisseau (12) conduisant l'urée par l'intermédiaire d'une sonde (28) ;
l'ouverture d'une valve (22) contrôlable, laquelle est disposée dans une conduite de fluide (24) se trouvant entre un réservoir (16) élastique et un dispositif d'occlusion (14) hydraulique, où l'ouverture se produit quand la pression mesurée par la sonde (28) dépasse une pression limite définie,
et dans lequel, quand la valve (22) est ouverte par le fait de la propre élasticité du réservoir (16) élastique, une circulation du fluide hydraulique se produit depuis le réservoir (16) élastique jusqu'au dispositif d'occlusion (14) hydraulique, jusqu'à ce que le même niveau de pression soit atteint tant dans le dispositif d'occlusion (14) que dans le réservoir (16), ou jusqu'à ce que la valve (22) contrôlable soit de nouveau fermée.
**caractérisé en ce que** la valve (22) contrôlable est entièrement ouverte quand la pression mesurée par la sonde (28) dépasse la pression limite, de telle sorte qu'un remplissage par à-coup du dispositif d'occlusion (14) hydraulique se produit avec un fluide hydraulique jusqu'à ce qu'un niveau de contrainte en pression suffisant soit atteint pour provoquer la fermeture en toute sécurité du vaisseau (12) conduisant l'urée pendant une situation de contrainte.

6. Procédé selon la revendication 5 **caractérisé en ce que**, après le remplissage par à-coup du dispositif d'occlusion (14) hydraulique, une partie du fluide hydraulique est de nouveau transportée jusque dans le réservoir (16) élastique par l'intermédiaire d'un dispositif de pompe (18), lequel est, en particulier, à commande électrique.

7. Procédé selon la revendication 6, **caractérisé en ce que** la pression dans le dispositif d'occlusion hydraulique est, en mode de fonctionnement normal, réduite à une pression standard qui est suffisante pour provoquer la fermeture en toute sécurité du vaisseau (12) conduisant l'urée.
